# EUROPEAN PATENT SPECIFICATION

(11) **EP 3 987 002 B1**
(45) Date of publication and mention of the grant of the patent: **10.01.2024**
(21) Application number: 20740719.8
(22) Date of filing: 16.06.2020
(51) Int. Cl.: C12M 3/00, C12M 1/22, C12M 1/00, C12M 1/36

(54) **DEVICE FOR CULTIVATING, CONTROLLING AND PROTECTING AN IN VITRO EMBRYO DEVELOPMENT**
VORRICHTUNG ZUM KULTIVIEREN, KONTROLLIEREN UND SCHÜTZEN EINER IN-VITRO-EMBRYOENTWICKLUNG
DISPOSITIF DE CULTURE, DE RÉGULATION ET DE PROTECTION D'UN DÉVELOPPEMENT EMBRYONNAIRE IN VITRO

(30) Priority: 21.06.2019 CH 8432019; 21.06.2019 IT 201900009843
(43) Date of publication of application: 27.04.2022
(73) Proprietor: IIARG International Institutes of Advanced Reproduction and Genetics SA, 6901 Lugano (CH); Descrovi, Aldo, 6839 Sagno (CH)
(72) Inventor: GIANAROLI, Luca, 6933 Muzzano (CH); MAGLI, Maria Cristina, 40050 Monte San Pietro (BO) (IT); DESCROVI, Aldo, 6839 Sagno (CH)
(74) Representative: Fabiano, Piero
(86) International application number: PCT/IB2020/055600
(87) International publication number: WO 2020/254950

(56) References cited:
- JP-A- 2000 093 156
- US-A1- 2018 282 682
- US-B2- 10 106 769

## Description

### Field of the invention

The present invention concerns the field of in vitro fertilization (IVF).

In particular, the present invention concerns a device for cultivating, controlling and protecting an in vitro embryo development.

### Known art

The levels of infertility have dramatically increased in the last decades, so much indeed that this phenomenon currently affects about 20% of couples, for whom the use of in vitro fertilization (IVF) is the only possibility of conceiving.

The current IVF techniques require numerous interventions by the biologists, including the direct manipulation of gametes, zygotes and embryos, as well as their regular monitoring, with a microscope, to control their morphology and development during the culture period. The extracorporeal step provides that the gametes and embryos are preserved in an incubator so that to maintain optimal conditions in terms of temperature, CO2 and humidity.

The fertilization of the oocyte and the development of the embryo strictly depend on the ability to keep the aforesaid parameters (temperature, CO2 and humidity) stable. In particular, repeated exposure to the outer environment during the manipulations can have severe consequences on the proper development of the embryo.

JP 2000 093156 discloses an incubation unit with a rotating shaft operating on a set of holders in a carousel.

EP2586858 describes a device for cultivating, controlling and protecting the automated development of an in vitro embryo so that to minimize exposure to the outer environment and contact with the operator of the embryo.

To this end, EP2586858 provides an anthropomorphous robot placed inside the incubator, the Applicant has however observed that the high humidity of the environment inside the incubator, around 95% on the average, can lead to an early deterioration of the mechanical parts of the robot with significant reduction of the device's reliability.

Moreover, the lubricants needed for the operation of the robot are not compatible with the required aseptic nature of the environment inside the incubator, thus making it difficult to implement the solution suggested in EP2586858.

The Applicant has thus addressed the problem of implementing a device for cultivating, controlling and protecting the development of an in vitro embryo which allows to reduce exposure to the outer environment and contact with the operator of the embryo to a minimum and which does not have the problems of the know art in terms of pollution of the environment inside the incubator and loss of reliability.

### Summary of the Invention

Thus, in its first aspect, the invention concerns a device for cultivating, controlling and protecting the development of an in vitro embryo, comprising:
- at least one incubation unit comprising at least one storing unit of the in vitro embryo;
- at least one inlet/outlet unit of biological material; said at least one inlet/outlet unit of biological material being in fluid communication with said incubation unit;
- at least one movement system for collecting a sample of biological material from said inlet/outlet unit and bringing it in least one storing unit of the in vitro embryo or vice-versa;
- said at least one movement system comprising: at least a base sleeve, at least one upright element integral with the base sleeve, and at least one head element comprising an extractable arm; the base sleeve being functionally connected to a first motor for rotating the upright element; the base sleeve and the first motor being placed outside at least one incubation unit. The upright element extending so that to enter the incubation unit; the incubation unit comprising a first sealing device at the inlet of the upright element into the incubation unit itself.

In the aforesaid aspect, the present invention can have at least one of the preferred characteristics defined hereunder.

Preferably, the base sleeve supports and is integral with the upright element so that a translation of the base sleeve involves a translation of the upright element.

Conveniently, the device for cultivating, controlling and protecting the development of an in vitro embryo further comprises a translating assembly to translate the upright element, comprising at least one sliding block integral with the base sleeve; at least one threaded shaft engaged with the sliding block so that the rotation of the threaded shaft involves the sliding of the sliding block along the threaded shaft. The threaded shaft being functionally connected to a second motor also arranged outside the incubation unit.

Preferably, the threaded shaft is functionally connected to a second motor by means of a transmission member comprising: a first pulley connected to a motor shaft of the second motor, a second pulley engaged to the threaded shaft and a transmission belt.

Preferably, the movement system comprises a first transmission assembly between the first motor and the upright element arranged outside said incubation unit, said transmission assembly being designed so that to transmit a rotary motion from the first motor to the upright element.

Conveniently, the first transmission assembly comprises: a third pulley engaged with the upright element, a fourth pulley constrained to a motor shaft of the first motor and a transmission belt extending between said third pulley and said fourth pulley.

Advantageously, the head element is a box-shaped element arranged inside the at least one incubation unit at an end of the upright element and integral therewith.

Preferably, the head element is a hermetically closed box-shaped element provided with an opening for the passage of the extractable arm; a sealing element being arranged between the aforesaid opening and the extractable arm.

Conveniently, the head element comprises a movement assembly of the extractable arm comprising: at least one guided pulley engaged to a rotating shaft; at least one driven pulley and a transmission belt extending between the guided pulley and the driven pulley, at least one constraining device to constrain said extractable arm to said transmission belt, the constraining device being shaped so that the rotation of the transmission belt in a direction involves the translation of the extractable arm in a direction and the rotation of the transmission belt in another direction involves the translation of the extractable arm in the opposite direction.

Preferably, the inlet/outlet unit of biological material comprises an elongated tubular body comprising: an inlet opening, an outlet opening and a conveyor belt extending inside the tubular body between the inlet opening and the outlet opening; the outlet opening putting the environment inside said tubular body in fluid communication with the environment inside the incubation unit, said inlet opening putting the environment inside the tubular body in communication with the environment outside the incubation unit.

Conveniently, the in vitro embryo is housed in a culture plate comprising an identification code detectable by a visual detection device.

Preferably, each storing unit of the culture plates comprises a plurality of shelves each shaped to support at least one culture plate.

Advantageously, the device for cultivating, controlling and protecting the development of an in vitro embryo comprises four storing units of the in vitro embryo, which are arranged inside the incubation unit along a circumference arranged concentrically to the upright element.

Preferably, the device for cultivating, controlling and protecting the development of an in vitro embryo can comprise at least one working unit with a microscope; the working unit with a microscope being positioned between the inlet opening of the inlet/outlet unit of the biological material and the incubation unit.

Further characteristics and advantages of the invention will become clearer in the detailed description of some preferred, but not exclusive, embodiments of a device for cultivating, controlling and protecting the development of an in vitro embryo according to the present invention.

### Brief description of the drawings

Such description will be set forth hereunder with reference to the accompanying drawings, only provided by way of example and thus not limiting, in which:
- figure 1 shows a partially sectional schematic side view of a device for cultivating, controlling and protecting the development of an in vitro embryo according to the present invention;
- figure 2 shows an always partially sectional schematic plan view of the device for cultivating, controlling and protecting the development of an in vitro embryo of figure 1;
- figure 3 shows an enlarged partially sectional schematic side view of an embodiment of the inlet/outlet unit of the biological material according to the present invention;
- figure 4 is a schematic side view of a first embodiment of the movement system according to present invention;
- figure 5 is a schematic top view of the head element of the movement system according to the present invention;
- figure 6 is a schematic rear view of the movement system shown in figure 4 according to the present invention;
- figure 7 is an enlarged schematic side view of an embodiment of a storing unit of the culture plates according to the present invention; and
- figure 8 is an enlarged schematic side view of an alternative embodiment of the inlet/outlet unit of biological material according to the present invention.

### Detailed description of embodiments of the invention

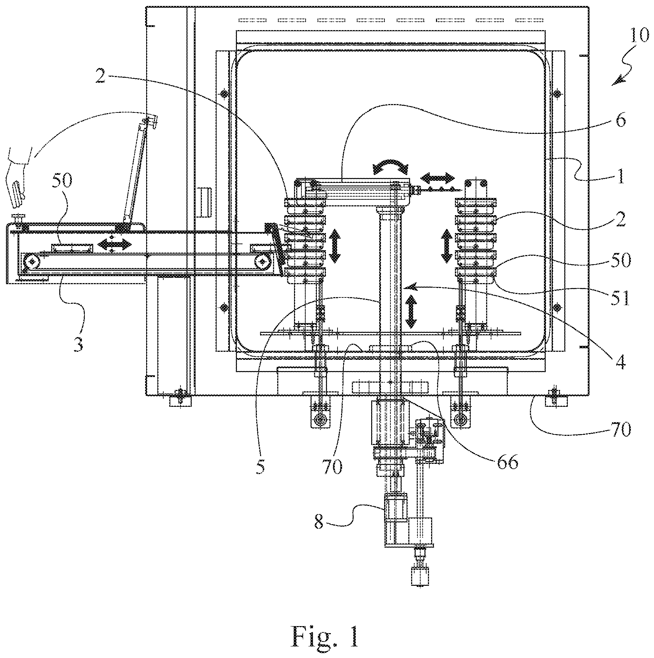

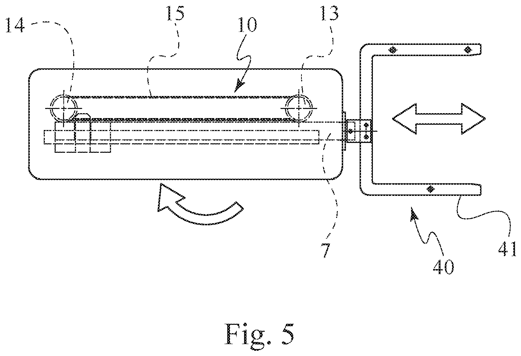

With reference to figures 1-8, a device for cultivating, controlling and protecting the development of an in vitro embryo according to the present invention is denoted by the numeral reference 10.

For ease of understanding, the devices and units are shown as if they were sectional in figures 1-8, in other words, also showing their inner components, although not visible from the outside.

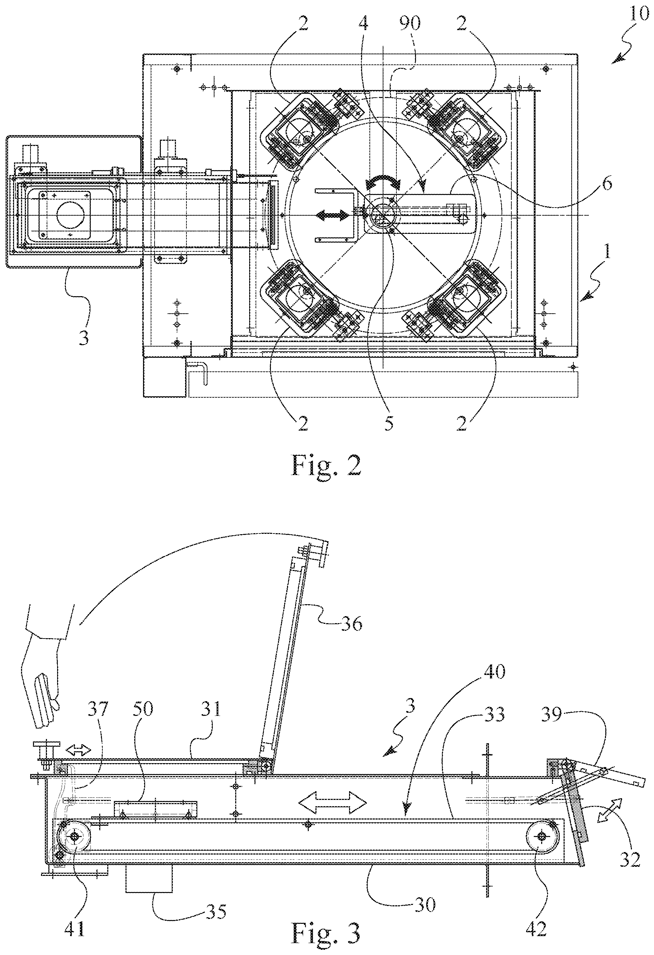

The device 10, as shown in figures 1 and 2, essentially consists of two modular units communicating with each other through a movement system and automatic openings which allow the transfer of culture plates 50 containing the biological material from one unit to another, without compromising the conditions of the controlled environment inside the device itself. The units composing the device 10 are essentially an incubation unit 1 and an inlet/outlet unit 3 of the biological material.

An enlarged embodiment of the inlet/outlet unit 3 of the biological material is shown in figure 3. The inlet/outlet unit 3 of the biological material is in fluid communication with the incubation unit 1. The inlet/outlet unit 3 has the primary task of allowing the operator to collect and insert the biological material from/into the device 100, and in particular from/into the incubation unit 1.

The inlet/outlet unit 3 of the biological material comprises an elongated tubular body 30 comprising an inlet opening 31, an outlet opening 32 and a conveyor belt 33 extending inside the tubular body 30 between the inlet opening 31 and the outlet opening 32.

The outlet opening 32 is designed to put the tubular body 30 in fluid communication with the inside of the incubation unit 1, whereas the inlet opening 31 is designed to put the inside of the tubular body 30 in communication with the environment outside the incubation unit 1.

The inlet opening 31 is closed by a door 36 and by an automated locking system which allows the opening of the door 36 for accessing the inlet opening 31 only in view of given parameters controlled by an appropriate control unit (not shown in the figures).

In specific, the locking system comprises: a lever 37, a control unit to control the lever 37 and a laser bar code reader 35, arranged and designed to detect the proper position and a code of a culture plate 50 placed on the conveyor belt 33.

The lever 37 is movable between a locked position of the door 36 and an unlocked position wherein the opening of the door 36 is allowed, thus the access to the inlet opening 31.

The laser bar code reader 35 detects and transmits the proper positioning of the culture plate 50 at the inlet opening 31 and the identification code of the culture plate 50 to the control unit.

Whenever the identification code of the plate placed at the opening 31 corresponds to that of the culture plate 50 required, the movement of the lever 37 towards the unlocked position is allowed, and consequently the unlocking of the door 36 to collect the culture plate 50 required.

Vice-versa, whenever the code read by the laser bar code reader 35 and transmitted to the control unit does not correspond to that of the culture plate 50 required, the opening of the door 36 would not be allowed and an alarm signal, preferably acoustic, would be sent.

A motorized door 39, always controlled by the control unit, is present at the outlet opening 32. The motor of the door 39, of the electric type, is connected to a gear train and opens and closes the door 39, under control of the control unit.

The control unit controls the opening of the door 36, only if it verified that the door 39 is closed and vice-versa.

In other words, the doors 36 and 39 cannot be opened simultaneously but, on the contrary, the opening of one of the two doors 36, 37 requires the other to stay closed.

The camera 50 is positioned outside the inlet/outlet unit 3 so that not to contaminate the environment inside the inlet/outlet unit 3 and consequently the environment inside the incubation unit 1.

The camera 50 captures the image through an inspection window obtained on a side wall of the elongated tubular body 30.

The conveyor belt 33 comprises a belt element 40, a first driven pulley 41 and a second pulley 42.

The first pulley 41 is rotated by a first motor, preferably electric. Similarly, the second pulley 42 is rotated by a second motor, also preferably of the electric type.

Both the first and second motors of the inlet/outlet unit 3 are driven by the control unit that also controls the doors 36, 39.

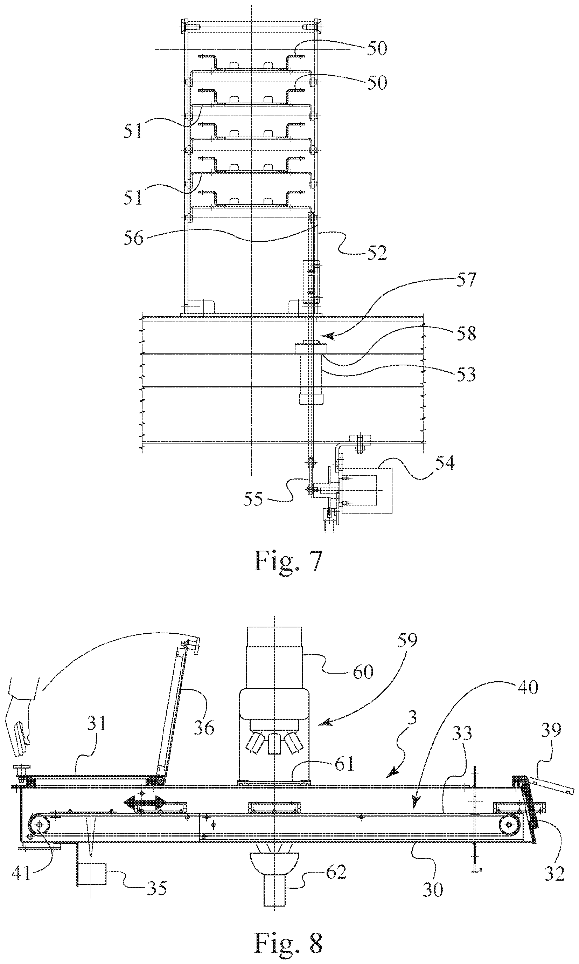

An alternative embodiment of the inlet/outlet unit 3 entirely similar to that of figure 3, except for the fact of having a microscope unit 59 interposed between the inlet opening 31 and the outlet opening 32, is shown in figure 8.

For ease of understanding, the common elements of the embodiments shown in figures 3 and 8 are denoted by the same numeral references.

The microscope unit 59 comprises: a microscope 60, an inspection window 61 arranged on a side wall of the tubular body 30, and a light source 62.

The incubation unit 1 is the core of the device and comprises at least one movement system 4, i.e. a robotic arm preferably cylindrical in shape, to transport the biological material, and at least one storing unit 2 for the culture plates 50. Preferably, each incubation unit 1 comprises, as shown in figure 2, four storing units 2 of the culture plates 50.

In the embodiment shown in figures 1 and 2, the storing units 2 are arranged inside the incubation unit 1 along a circumference 90 arranged concentrically to the movement system 4.

Each storing unit 2 is provided with a plurality of shelves 51 each shaped to support at least one culture plate 50.

Figure 7 shows an enlarged side view of an embodiment of a storing unit 2.

The storing unit 2 shown in figure 7 comprises a plurality of shelves 51 each shaped to house a culture plate 50, the shelves 51 are held up by a vertical supporting structure 52 so that to be mutually spaced and arranged in a corresponding position.

A swinging device 57, shaped and designed to swing each shelf 51 and consequently each culture plate 50 positioned therein, to imitate the movements of the maternal womb, can be provided for each storing unit 2.

The swinging device 57 comprises an arm 56 extending vertically to engage an end of each shelf 51.

The arm 56 is connected to an electric motor 54 through a rod crank system 55, the rod crank system 55 transforms the rotary motion of the electric motor 54 into a vertical translation of the arm 56.

The electric motor 54 is arranged outside the incubation unit 1 to avoid the contamination of the environment inside the incubation unit 1.

To this end, the swinging device 57 comprises a sealing member 53 arranged on the arm 56 at the opening 58 made on the bottom wall 70 of the incubation unit 1. The arm 56 penetrates into the incubation unit 1 through the opening 58 to reach the shelves 51.

The movement system 4 has the task of collecting the culture plates 50 from the tubular body 30 and of placing them in the appropriate storing units 2 and vice-versa.

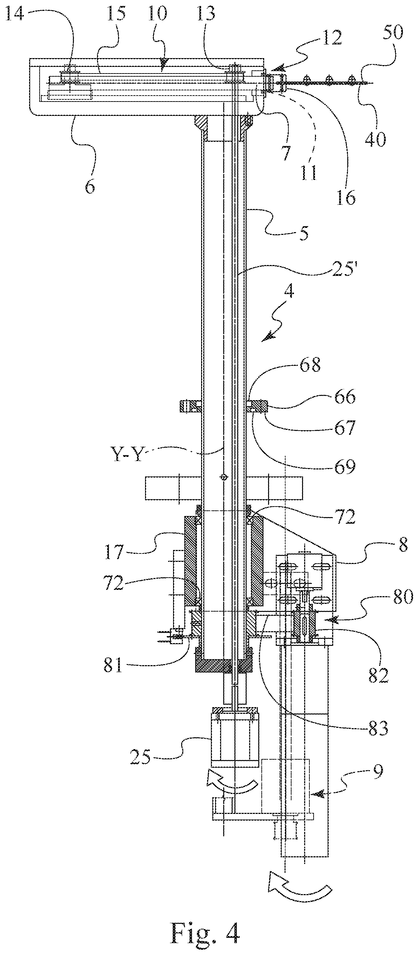

To this end, the movement system 4, shown in more detail in figures 4-6, comprises: at least one base sleeve 17, at least one upright element 5 integral with the base sleeve 17, and at least one head element 6.

The head element 6 in turn has an extractable arm 7.

The base sleeve 17 is functionally connected to a first motor 8 for rotating the upright element 5. The base sleeve 17 and the first motor 8 are placed outside the incubation unit 1 so that not to contaminate the environment inside the incubation unit 1 and so that to not be subjected to the humidity of the environment inside the latter.

The upright element 5 extends vertically, along a vertical axis Y-Y, at least between the base sleeve 17 and the head element 6 so that to enter the incubation unit 1. Preferably, the upright element 5 also extends downwards beyond the base sleeve 17.

The incubation unit 1 has a first hermetic sealing device 66 at the inlet of the upright element 5 in the incubation unit 1 itself.

The first sealing device 66 is made with an annular element 67 preferably made of steel and shaped to be mounted on the upright element 5. To this end, the annular element 67 has a through opening of size and shape substantially corresponding to that of the upright element 5.

The annular element 67 further has a first seat 68 apt to accommodate a first gasket, such as an o-ring, which is sealingly arranged between the upright element 5 and the annular element 67, and a second seat 69 apt to accommodate a second gasket, such as an o-ring, which is sealingly arranged between the annular element 67 and the bottom wall 70 of the incubation unit 1. A first transmission assembly 80, also arranged outside the incubation unit 1, is present to transmit the rotary motion between the first motor 8 and the upright element 5.

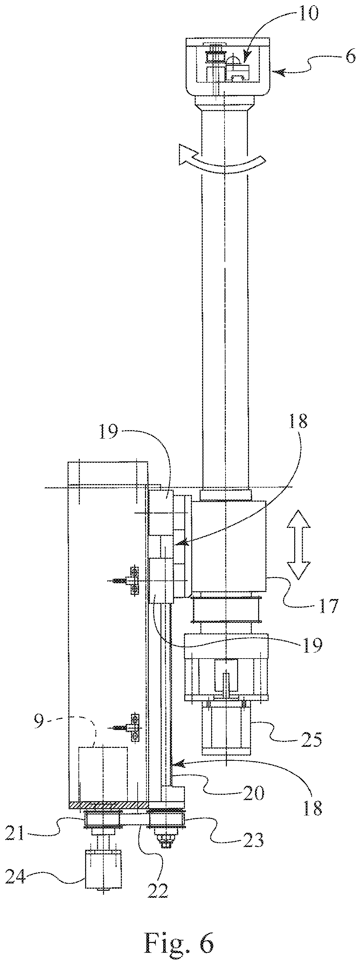

With reference to the embodiment shown in figures 4 and 6, the first transmission assembly 80 is arranged under the base sleeve 17. The first transmission assembly 80 is provided with a third pulley 81 engaged with the upright element 5, a fourth pulley 82 engaged with the motor shaft of the first motor 8 and a transmission belt 83 extending between the two pulleys 81, 82.

The base sleeve 17 is integral with and supports the upright element 5 through two bearings 72, so that a translation of the base sleeve 17 involves a translation of the upright element 5.

By translating vertically along a direction parallel to the axis Y-Y, the upright element 5 translates the head element 6 placed on its top. An appropriate translation assembly 18 is provided to translate the upright element 5 and, consequently, the head element 6.

The translation assembly 18 of the upright element 5 comprises: at least one sliding block 19, preferably two, integral with the base sleeve 17, and at least one threaded shaft 20 engaged with the sliding blocks 19 so that the rotation of the threaded shaft 20 involves the sliding of the sliding blocks 19 along the threaded shaft 20 itself.

The threaded shaft 20 is functionally connected to a second motor 9 by means of a transmission member. The transmission member is also arranged outside the incubation unit 1.

The transmission member consists of a first pulley 21 connected to the motor shaft of the second motor 9, a second pulley 23 engaged to the threaded shaft 20, and a transmission belt 22.

Moreover, there is an encoder 24 configured to detect the revolutions of the second motor 9 and, consequently, to determine the vertical positioning of the base sleeve 17 with respect to the threaded shaft 20.

Always with reference to figure 4, the head element 6 is a box-shaped element entirely arranged inside the incubation unit 1. The head element 6 is positioned at an end of the upright element 5 and is rotatably supported around the vertical axis Y-Y by the latter.

The head element 6 is a hermetically closed box-shaped element which contains the extractable arm 7 and its movement assembly 10 so that to protect the environment inside the incubation unit 1 from possible contaminations.

To this end, the head element 6 is made of a nontoxic material, for example steel or acetate or a combination thereof.

In the embodiment shown in the figures, the head element 6 comprises a closed upper door, which allows, by removing it, to inspect the movement assembly 10, and an opening 11 for the passage of the extractable arm 7. A second sealing element 12, such as a gasket or o-ring, is arranged between the opening 11 and the extractable arm 7.

The movement assembly 10 of the extractable arm comprises at least one guided pulley 13 engaged with a rotating shaft 25', an idler pulley 14 and a transmission belt 15 extending from the guided pulley 13 to the idler pulley 14.

The movement assembly 10 further comprises a constraining device to constrain the extractable arm 7 to the transmission belt 15. The device being shaped so that the rotation of the transmission belt 15 in a direction involves the translation of the arm 7 in a direction, for example the forward translation, and the rotation of the transmission belt 15 in another direction involves the translation of the extractable arm 7 in the opposite direction.

The rotating shaft 25' extends throughout the upright element 5 and is engaged in a motor 25 also arranged outside the incubation unit 1.

The motor 25, conveniently controlled by a control unit, is responsible, through the rotating shaft 25', of the rotation of the guided pulley 13 and, consequently, of the translation of the extractable arm 7 in a direction or in the opposite direction.

The extractable arm 7 has an end 16 which stays outside the box-shaped body, such end 16 supports a grasping member 40.

In the embodiment shown in figures 4-5, the grasping member 40 is a fork 41 able to couple with appropriate gripping surfaces for the culture plates 50.

The biological material needed for the in vitro fertilization process is in fact contained in appropriate culture plates 50 made of polymeric material. Such plates were appropriately designed for the purpose. It is however possible to use conventional plates currently on the market.

Although explicit reference was made in the present description to the use of the device object of the present invention, specifically intended for cultivating, controlling and protecting the development of an in vitro embryo, it is clear that the same device can be used for cultivating other biological material, such as for example stem cells, without departing from the protection scope of the present invention.

Several changes can be made to the embodiments described in detail, anyhow remaining within the protection scope of the invention, defined by the following claims.

## Claims

1. Device (10) for cultivating, controlling and protecting an in vitro embryo development, comprising:
- at least one incubation unit (1) comprising at least one storing unit (2) of the in vitro embryo;
- at least one inlet/outlet unit (3) of biological material; said at least one inlet/outlet unit (3) of biological material being in fluid communication with said incubation unit (1);
- at least one movement system (4) for collecting a sample of biological material from said inlet/outlet unit and bringing it in least one storing unit (2) of the in vitro embryo or vice-versa;
- said at least one movement system (4) comprising: at least a base sleeve (17), at least one upright element (5) integral with said base sleeve (17), and at least one head element (6) comprising an extractable arm (7); said base sleeve (17) being functionally connected to a first motor (8) for rotating said upright element (5); said base sleeve (17) and said first motor (8) being placed outside said at least one incubation unit (1) and said upright element (5) extending so that to enter said incubation unit (1); said incubation unit (1) comprising a first sealing device (66) at the inlet of the upright element into the incubation unit (1) itself.

2. Device (10) for cultivating, controlling and protecting an in vitro embryo development according to claim 1, **characterized in that** said base sleeve (17) is translatable, supports and is integral with said upright element (5) so that a translation of said base sleeve (17) involves a translation of said upright element (5).

3. Device for cultivating, controlling and protecting an in vitro embryo development according to claim 2, **characterized by** comprising a translating assembly (18) to translate said upright element (5) comprising at least one sliding block (19) integral with said base sleeve (17); at least one threaded shaft (20) engaged with said at least one sliding block (19) so that the rotation of the threaded shaft (20) involves the sliding of said at least one sliding block (19) along said threaded shaft (20), said threaded shaft (20) being functionally connected to a second motor (9) arranged outside said incubation unit (1).

4. Device for cultivating, controlling and protecting an in vitro embryo development according to claim 1, **characterized in that** said threaded shaft (20) is functionally connected to a second motor (9) by means of a transmission member comprising a first pulley (21) connected to a motor shaft of said second motor (9), a second pulley (23) engaged to said threaded shaft (20) and a transmission belt (22).

5. Device for cultivating, controlling and protecting an in vitro embryo development according to claim 1, **characterized by** comprising a first transmission assembly (80) between said first motor (8) and said upright element (5) arranged outside said incubation unit (1), said transmission assembly (80) being designed so that to transmit a rotary motion from said first motor (8) to said upright element (5).

6. Device for cultivating, controlling and protecting an in vitro embryo development according to claim 5, **characterized in that** said first transmission assembly (80) comprises: a third pulley (81) engaged with said upright element (5), a fourth pulley (82) constrained to a motor shaft of said first motor (8) and a transmission belt (83) extending between said third pulley (81) and said fourth pulley (82).

7. Device for cultivating, controlling and protecting an in vitro embryo development according to claim 1, **characterized in that** said head element (6) is a box-shaped element arranged inside said at least one incubation unit (1) at an end of said upright element (5) and integral therewith.

8. Device for cultivating, controlling and protecting an in vitro embryo development according to claim 7, **characterized in that** said head element (6) is a hermetically closed box-shaped element provided with an opening (11) for the passage of said extractable arm (7), a sealing element (12) being arranged between said opening (11) and said extractable arm (7).

9. Device for cultivating, controlling and protecting an in vitro embryo development according to claim 1, **characterized in that** said head element (6) comprises a movement assembly (10) of the extractable arm (7) comprising: at least one guided pulley (13) engaged to a rotating shaft (25'); at least one driven pulley (14) and a transmission belt (15) extending between the guided pulley (13) and the driven pulley (14), at least one constraining device to constrain said extractable arm (7) to said transmission belt (15), the constraining device being shaped so that the rotation of the transmission belt (15) in a direction involves the translation of the extractable arm (7) in a direction and the rotation of the transmission belt (15) in another direction involves the translation of the extractable arm (7) in the opposite direction.

10. Device for cultivating, controlling and protecting an in vitro embryo development according to claim 1, **characterized in that** said at least one inlet/outlet unit (3) of biological material comprises an elongated tubular body (30) comprising: an inlet opening (31), an outlet opening (32) and a conveyor belt (33) extending inside the tubular body (30) between said inlet opening (31) and said outlet opening (32); said outlet opening (32) putting the environment inside said tubular body (30) in fluid communication with the environment inside the incubation unit (1), said inlet opening (31) putting the environment inside the tubular body (30) in communication with the environment outside the incubation unit (1).

11. Device for cultivating, controlling and protecting an in vitro embryo development according to claim 1, **characterized in that** said in vitro embryo is housed in a culture plate (50) comprising an identification code detectable by a visual detection device (35).

12. Device for cultivating, controlling and protecting an in vitro embryo development according to claim 11, **characterized in that** each storing unit (2) of the culture plates (50) comprises a plurality of shelves (51) each shaped to support at least one culture plate (50).

13. Device for cultivating, controlling and protecting an in vitro embryo development according to claim 1, **characterized by** comprising four storing units (2) of the in vitro embryo, which are arranged inside the incubation unit (1) along a circumference (90) arranged concentrically to said upright element (5).

14. Device for cultivating, controlling and protecting an in vitro embryo development according to claim 1, **characterized by** comprising at least one working unit with a microscope (59); said working unit with a microscope (59) being positioned between said inlet opening (31) of said inlet/outlet unit (3) of the biological material and said incubation unit (1).

## Patentansprüche

1. Vorrichtung (10) zum Kultivieren, Kontrollieren und Schützen einer In-vitro-Embryoentwicklung, umfassend:
- mindestens eine Inkubationseinheit (1) umfassend mindestens eine Lagerungseinheit (2) für den in vitro Embryo;
- mindestens eine Einlass-/Auslass-Einheit (3) für biologisches Material; wobei die mindestens eine Einlass-/Auslasseinheit (3) für biologisches Material mit der Inkubationseinheit (1) in fluidischer Verbindung steht;
- mindestens ein Bewegungssystem (4) zur Entnahme einer Probe biologischen Materials aus der Einlass-/Auslasseinheit und zum Einbringen derselben in mindestens eine Lagerungseinheit (2) für den In-vitro-Embryo oder umgekehrt;
- wobei das mindestens eine Bewegungssystem (4) umfasst: mindestens eine Basishülse (17), mindestens ein mit der Basishülse (17) einstückig verbundenes Ständerelement (5) und mindestens ein Kopfelement (6) mit einem ausziehbaren Arm (7); wobei die Basishülse (17) mit einem ersten Motor (8) zur Drehung des Ständerelements (5) funktional verbunden ist; wobei die Basishülse (17) und der erste Motor (8) außerhalb der mindestens einen Inkubationseinheit (1) angeordnet sind und das Ständerelement (5) sich derart erstreckt, dass es in die Inkubationseinheit (1) eindringt; wobei die Inkubationseinheit (1) eine erste Dichtungsvorrichtung (66) am Einlass des Ständerelements in die Inkubationseinheit (1) aufweist.

2. Vorrichtung (10) zum Kultivieren, Kontrollieren und Schützen einer In-vitro-Embryoentwicklung gemäß Anspruch 1, **dadurch gekennzeichnet, dass** die Basishülse (17) verfahrbar ist, das Ständerelement (5) trägt und einstückig mit diesem verbunden ist, sodass eine Verschiebung der Basishülse (17) eine Verschiebung des Ständerelements (5) bewirkt.

3. Vorrichtung zum Kultivieren, Kontrollieren und Schützen einer In-vitro-Embryoentwicklung gemäß Anspruch 2, **dadurch gekennzeichnet, dass** sie eine zur Verschiebung des Ständerelements (5) dienende Translationseinheit (18) umfasst, die mindestens einen mit der Basishülse (17) einstückig verbundenen Gleitblock (19) aufweist; mindestens eine Gewindewelle (20), die mit dem mindestens einen Gleitblock (19) in Eingriff steht, so dass die Drehung der Gewindewelle (20) das Gleiten des mindestens einen Gleitblocks (19) entlang der Gewindewelle (20) bewirkt, wobei die Gewindewelle (20) funktionell mit einem zweiten Motor (9) verbunden ist, der außerhalb der Inkubationseinheit (1) angeordnet ist.

4. Vorrichtung zum Kultivieren, Kontrollieren und Schützen einer In-vitro-Embryoentwicklung gemäß Anspruch 1, **dadurch gekennzeichnet, dass** die Gewindewelle (20) funktionell mit einem zweiten Motor (9) mittels eines Getriebeelements verbunden ist, das eine erste Riemenscheibe (21), die mit einer Motorwelle des zweiten Motors (9) verbunden ist, eine zweite Riemenscheibe (23), die mit der Gewindewelle (20) in Eingriff steht, und einen Übertragungsriemen (22) umfasst.

5. Vorrichtung zum Kultivieren, Kontrollieren und Schützen einer In-vitro-Embryoentwicklung gemäß Anspruch 1, **dadurch gekennzeichnet, dass** sie eine zwischen dem ersten Motor (8) und dem Ständerelement (5) angeordnete erste Getriebeanordnung (80) umfasst, die außerhalb der Inkubationseinheit (1) angeordnet ist, wobei die Getriebeanordnung (80) so ausgelegt ist, dass sie eine Drehbewegung von dem ersten Motor (8) auf das aufrechte Element (5) überträgt.

6. Vorrichtung zum Kultivieren, Kontrollieren und Schützen einer In-vitro-Embryoentwicklung gemäß Anspruch 5, **dadurch gekennzeichnet, dass** die erste Getriebeanordnung (80) Folgendes umfasst: eine dritte Riemenscheibe (81), die mit dem Ständerelement (5) in Eingriff steht, eine vierte Riemenscheibe (82), die mit einer Motorwelle des ersten Motors (8) verbunden ist, und einen Übertragungsriemen (83), der sich zwischen der dritten Riemenscheibe (81) und der vierten Riemenscheibe (82) erstreckt.

7. Vorrichtung zum Kultivieren, Kontrollieren und Schützen einer In-vitro-Embryoentwicklung gemäß Anspruch 1, **dadurch gekennzeichnet, dass** das Kopfelement (6) einkastenförmiges Element ist, das innerhalb der mindestens einen Inkubationseinheit (1) an einem Ende des Ständerelements (5) angeordnet und mit diesem einstückig verbunden ist.

8. Vorrichtung zum Kultivieren, Kontrollieren und Schützen einer In-vitro-Embryoentwicklung gemäß Anspruch 7, **dadurch gekennzeichnet, dass** das Kopfelement (6) ein hermetisch geschlossenes, kastenförmiges Element ist, das mit einer Öffnung (11) für den Durchgang des ausziehbaren Arms (7) versehen ist, wobei ein Dichtelement (12) zwischen der Öffnung (11) und dem ausziehbaren Arm (7) angeordnet ist.

9. Vorrichtung zum Kultivieren, Kontrollieren und Schützen einer In-vitro-Embryoentwicklung gemäß Anspruch 1, **dadurch gekennzeichnet, dass** das Kopfelement (6) eine Bewegungsanordnung (10) des ausziehbaren Arms (7) aufweist, umfassend: mindestens eine geführte Riemenscheibe (13), die mit einer Drehwelle (25') verbunden ist; mindestens eine angetriebene Riemenscheibe (14) und einen Übertragungsriemen (15), der sich zwischen der geführten Riemenscheibe (13) und der angetriebenen Riemenscheibe (14) erstreckt, mindestens eine Verbindungsvorrichtung, um den ausziehbaren Arm (7) mit dem Übertragungsriemen (15) zu verbinden, wobei die Verbindungsvorrichtung so geformt ist, dass die Drehung des Übertragungsriemens (15) in einer Richtung die Verschiebung des ausziehbaren Arms (7) in einer Richtung und die Drehung des Übertragungsriemens (15) in einer anderen Richtung die Verschiebung des ausziehbaren Arms (7) in der entgegengesetzten Richtung bewirkt.

10. Vorrichtung zum Kultivieren, Kontrollieren und Schützen einer In-vitro-Embryoentwicklung gemäß Anspruch 1, **dadurch gekennzeichnet, dass** die mindestens eine Einlass-/Auslasseinheit (3) für biologisches Material einen länglichen rohrförmigen Körper (30) aufweist, umfassend: eine Einlassöffnung (31), eine Auslassöffnung (32) und ein Förderband (33), das sich im Inneren des rohrförmigen Körpers (30) zwischen der Einlassöffnung (31) und der Auslassöffnung (32) erstreckt; wobei die Auslassöffnung (32) die Umgebung im Inneren des rohrförmigen Körpers (30) in Fluidverbindung mit der Umgebung im Inneren der Inkubationseinheit (1) bringt und die Einlassöffnung (31) die Umgebung im Inneren des rohrförmigen Körpers (30) in Verbindung mit der Umgebung außerhalb der Inkubationseinheit (1) bringt.

11. Vorrichtung zum Kultivieren, Kontrollieren und Schützen einer In-vitro-Embryoentwicklung gemäß Anspruch 1, **dadurch gekennzeichnet, dass** der In-vitro-Embryo in einer Kulturplatte (50) aufgenommen ist, die einen Identifikationscode aufweist, der durch eine visuelle Erfassungsvorrichtung (35) erkennbar ist.

12. Vorrichtung zum Kultivieren, Kontrollieren und Schützen einer In-vitro-Embryoentwicklung gemäß Anspruch 11, **dadurch gekennzeichnet, dass** jede Lagerungseinheit (2) der Kulturplatten (50) eine Vielzahl von Ablagen (51) umfasst, die jeweils zur Lagerung mindestens einer Kulturplatte (50) ausgebildet sind.

13. Vorrichtung zum Kultivieren, Kontrollieren und Schützen einer In-vitro-Embryoentwicklung gemäß Anspruch 1, **dadurch gekennzeichnet, dass** sie vier Lagerungseinheiten (2) des In-vitro-Embryos umfasst, die im Inneren der Inkubationseinheit (1) entlang eines konzentrisch zu dem Ständerelement (5) angeordneten Umfangs (90) angeordnet sind.

14. Vorrichtung zum Kultivieren, Kontrollieren und Schützen einer In-vitro-Embryoentwicklung gemäß Anspruch 1, **dadurch gekennzeichnet, dass** sie mindestens eine Arbeitseinheit mit einem Mikroskop (59) umfasst, wobei die Arbeitseinheit mit einem Mikroskop (59) zwischen der Einlassöffnung (31) der Einlass-/Auslasseinheit (3) für das biologische Material und der Inkubationseinheit (1) angeordnet ist.

## Revendications

1. Dispositif (10) pour cultiver, contrôler et protéger un développement embryonnaire in vitro, comprenant:
- au moins une unité d'incubation (1) comprenant au moins une unité de stockage (2) de l'embryon in vitro;
- au moins une unité d'entrée/de sortie (3) de matériel biologique; ladite au moins une unité d'entrée/de sortie (3) de matériel biologique étant en communication fluidique avec ladite unité d'incubation (1);
- au moins un système de mouvement (4) pour prélever un échantillon de matériel biologique de ladite unité d'entrée/de sortie et l'amener dans au moins une unité de stockage (2) de l'embryon in vitro ou vice versa;
- ledit au moins un système de mouvement (4) comprenant au moins un manchon de base (17), au moins un élément montant (5) intégral avec ledit manchon de base (17) et au moins un élément de tête (6) comprenant un bras extractible (7); ledit manchon de base (17) étant fonctionnellement relié à un premier moteur (8) pour faire tourner ledit élément montant (5); ledit manchon de base (17) et ledit premier moteur (8) étant placés en dehors de ladite unité d'incubation (1) et ledit élément montant (5) s'étendant de sorte à entrer dans ladite unité d'incubation (1); ladite unité d'incubation (1) comprenant un premier dispositif d'étanchéité (66) en correspondance de l'entrée de l'élément montant dans l'unité d'incubation (1) elle-même.

2. Dispositif (10) pour cultiver, contrôler et protéger un développement embryonnaire in vitro selon la revendication 1, **caractérisé en ce que** ledit manchon de base (17) peut être translaté, supporte et est solidaire avec ledit élément montant (5) de sorte qu'une translation dudit manchon de base (17) implique une translation dudit élément montant (5).

3. Dispositif pour cultiver, contrôler et protéger un développement embryonnaire in vitro selon la revendication 2, **caractérisé en ce qu'**il comprend un ensemble de translation (18) pour translater ledit élément montant (5) comprenant au moins un bloc coulissant (19) solidaire avec ledit manchon de base (17); au moins un arbre fileté (20) étant engagé avec ledit au moins un bloc coulissant (19) de sorte que la rotation de l'arbre fileté (20) implique le coulissement dudit au moins un bloc coulissant (19) le long dudit arbre fileté (20), ledit arbre fileté (20) étant fonctionnellement relié à un second moteur (9) disposé en dehors de ladite unité d'incubation (1).

4. Dispositif pour cultiver, contrôler et protéger un développement embryonnaire in vitro selon la revendication 1, **caractérisé en ce que** ledit arbre fileté (20) est fonctionnellement relié à un second moteur (9) au moyen d'un élément de transmission comprenant une première poulie (21) reliée à un arbre moteur dudit second moteur (9), une seconde poulie (23) engagée avec ledit arbre fileté (20) et une courroie de transmission (22).

5. Dispositif pour cultiver, contrôler et protéger un développement embryonnaire in vitro selon la revendication 1, **caractérisé en ce qu'**il comprend un premier ensemble de transmission (80) entre ledit premier moteur (8) et ledit élément montant (5) disposé en dehors de l'unité d'incubation (1), ledit ensemble de transmission (80) étant conçu de sorte à transmettre un mouvement de rotation dudit premier moteur (8) audit élément montant (5).

6. Dispositif pour cultiver, contrôler et protéger un développement embryonnaire in vitro selon la revendication 5, **caractérisé en ce que** ledit premier ensemble de transmission (80) comprend: une troisième poulie (81) engagée avec ledit élément montant (5), une quatrième poulie (82) contrainte à un arbre moteur dudit premier moteur (8) et une courroie de transmission (83) s'étendant entre ladite troisième poulie (81) et ladite quatrième poulie (82).

7. Dispositif pour cultiver, contrôler et protéger un développement embryonnaire in vitro selon la revendication 1, **caractérisé en ce que** ledit élément de tête (6) est un élément en forme de boîte disposé à l'intérieur de ladite au moins une unité d'incubation (1) en correspondance d'une extrémité dudit élément montant (5) et solidaire avec celui-ci.

8. Dispositif pour cultiver, contrôler et protéger un développement embryonnaire in vitro selon la revendication 7, **caractérisé en ce que** ledit élément de tête (6) est un élément en forme de boîte hermétiquement fermé et pourvu d'une ouverture (11) pour le passage dudit bras extractible (7), un élément d'étanchéité (12) étant disposé entre ladite ouverture (11) et ledit bras extractible (7).

9. Dispositif pour cultiver, contrôler et protéger un développement embryonnaire in vitro selon la revendication 1, **caractérisé en ce que** ledit élément de tête (6) comprend un ensemble de mouvement (10) du bras extractible (7) et comprenant: au moins une poulie guidée (13) engagée avec un arbre rotatif (25'); au moins une poulie guidée (14) et une courroie de transmission (15) s'étendant entre la poulie guidée (13) et la poulie entraînée (14), au moins un dispositif de contrainte pour contraindre ledit bras extractible (7) à ladite courroie de transmission (15), le dispositif de contrainte étant conformé de sorte à ce que la rotation de courroie de transmission (15) dans une direction implique la translation du bras extractible (7) dans une direction et que la rotation de la courroie de transmission (15) dans une autre direction implique la translation du bras extractible (7) dans la direction opposée.

10. Dispositif pour cultiver, contrôler et protéger un développement embryonnaire in vitro selon la revendication 1, **caractérisé en ce que** l'au moins une unité d'entrée/de sortie (3) de matériel biologique comprend un corps tubulaire allongé (30) comprenant: une ouverture d'entrée (31), une ouverture de sortie (32) et une bande transporteuse (33) s'étendant à l'intérieur du corps tubulaire (30) entre ladite ouverture d'entrée (31) et ladite ouverture de sortie (32); ladite ouverture de sortie (32) mettant l'environnement à l'intérieur dudit corps tubulaire (30) en communication fluidique avec l'environnement à l'intérieur de l'unité d'incubation (1), ladite ouverture d'entrée (31) mettant l'environnement à l'intérieur du corps tubulaire (30) en communication avec l'environnement à l'extérieur de l'unité d'incubation (1).

11. Dispositif pour cultiver, contrôler et protéger un développement embryonnaire in vitro selon la revendication 1, **caractérisé en ce que** ledit embryon in vitro est logé dans une plaque de culture (50) comprenant un code d'identification détectable par un dispositif de détection visuelle (35).

12. Dispositif pour cultiver, contrôler et protéger un développement embryonnaire in vitro selon la revendication 11, **caractérisé en ce que** chaque unité de stockage (2) des plaques de culture (50) comprend une pluralité d'étagères (51) chacune conformée pour supporter au moins une plaque de culture (50).

13. Dispositif pour cultiver, contrôler et protéger un développement embryonnaire in vitro selon la revendication 1, **caractérisé en ce qu'**il comprend quatre unités de stockage (2) de l'embryon in vitro, qui sont disposées à l'intérieur de l'unité d'incubation (1) le long d'une circonférence (90) disposée concentriquement audit élément montant (5).

14. Dispositif pour cultiver, contrôler et protéger un développement embryonnaire in vitro selon la revendication 1, **caractérisé en ce qu'**il comprend au moins une unité de travail avec un microscope (59); ladite unité de travail avec un microscope (59) étant positionnée entre ladite ouverture d'entrée (31) et ladite unité d'entrée/de sortie (3) du matériel biologique et ladite unité d'incubation (1).
